# FASCICULE DE BREVET EUROPEEN

(11) **EP 1 418 941 B1**
(45) Date de publication et mention de la délivrance du brevet: **07.07.2010**
(21) Numéro de dépôt: 02740841.8
(22) Date de dépôt: 29.05.2002
(51) Int. Cl.: A61K 39/21, C07K 14/155, C12N 15/863

(54) **INDUCTION DE REPONSE IMMUNITAIRE CONTRE LE VIRUS DE L'IMMUNODEFICIENCE FELINE**
INDUZIERTE IMMUNANTWORT GEGEN DEN KATZEN-IMMUNSCHWÄCHE-VIRUS
INDUCTED IMMUNE RESPONSE AGAINST THE FELINE IMMUNODEFICIENCY VIRUS

(30) Priorité: 01.06.2001 FR 0107275
(43) Date de publication de la demande: 19.05.2004
(73) Titulaire: MERIAL, 69007 Lyon (FR)
(72) Inventeur: FISCHER, Laurent, F-69110 Sainte Foy Les Lyon (FR)
(74) Mandataire: Nargolwalla, Cyra
(86) Numéro de dépôt international: PCT/FR2002/001809
(87) Numéro de publication internationale: WO 2002/096349

(56) Documents cités:
- EP-A- 1 074 625
- WO-A-00/77043
- WO-A-95/30019
- WO-A-98/21354
- I. RAMSHAW ET AL.: "The prime-boost strategy: exciting prospects for improved vaccination" IMMUNOLOGY TODAY, vol. 21, no. 4, 2000, pages 163-165, XP002193913
- RICHARDSON J.: 'ENHANCEMENT OF FELINE IMMUNODEFICIENCY VIRUS (FIV) INFECTION AFTER DNA VACCINATION WITH THE FIV ENVELOPE' JOURNAL OF VIROLOGY vol. 7, no. 12, Décembre 1997, pages 9640 - 9649, XP000944623
- BORETTI F.S. ET AL: 'PROTECTION AGAINST FIV CHALLENGE INFECTION BY GENETIC VACCINATION USING MINIMALISTIC DNA CONSTRUCTS FOR FIV ENV GENE AND FELINE IL-12 EXPRESSION' AIDS vol. 14, no. 12, 18 Août 2000, pages 1749 - 1757
- KENT STEPHEN J. ET AL: 'Enhanced T-cell immunogenicity and protective immunodeficiency virus type 1 vaccine regimen consisting of consecutive priming with DNA and boosting with recombinant fowlpox virus' JOURNAL OF VIROLOGY, vol. 72, no. 12, Décembre 1998, pages 10180 - 10188, XP002179215

## Description

La présente invention a pour objet l'immunisation induction d'une réponse immunitaire contre le virus de l'immunodéficience féline. Elle a également pour objet des kits d'immunisation.

Le virus de l'immunodéficience féline (FIV) est un rétrovirus à ARN simple brin de polarité positive appartenant à la famille des lentivirus.

Largement répandu sur l'ensemble de la planète, le FIV touche essentiellement les félins, en particulier les chats.

La maladie se caractérise par une détérioration et une suppression du système immunitaire permettant le développement de maladies opportunistes et pouvant conduire à la mort.

La molécule d'ARN de FIV se compose de plusieurs cadres ouverts de lecture (COLs) codant pour les protéines de structure, notamment env et gag, les enzymes virales, notamment pol et pro, les transactivateurs, notamment tat et rev. Le COL rev est composé de deux exons.

Des vaccins inactivés ont été proposés, mais nécessitent pour être protecteurs des quantités d'antigènes très importantes ce qui pose des problèmes d'industrialisation.

D'autres approches vaccinales ont été tentées en particulier l'utilisation de la protéine d'enveloppe sous forme de sous-unité ou exprimée par un vecteur d'expression recombiné. Ces travaux n'ont pas abouti à des résultats satisfaisants. Ils ont par ailleurs mis en évidence des phénomènes de facilitation se traduisant par une virémie plus précoce chez les animaux vaccinés que chez les animaux témoins.

L'article de Cuisinier (Cuisinier A. M. et al., Vaccine, 1997, 15(10), 1085-1094) rappelle que suite à des essais de vaccination à l'aide de virus inactivés il est apparu que la glycoprotéine de surface gp120 serait déterminante pour la protection. Il rappelle toutefois que plusieurs essais de vaccination utilisant la protéine env rencombinée n'ont pas permis d'induire une protection. Les auteurs décrivent des expériences de vaccination de chats par administration intramusculaire d'ADN codant pour gp120 et p10 (nucléocapside) de FIV. L'administration d'ADN codant pour gp120 de FIV n'induit qu'une protection partielle. En cas de combinaison gp120 et p10 on n'observe pas de protection.

De son côté l'article de Richardson (Richardson J. et al., J. Virol., 1997, 71(12), 9640-9649) rapporte avec l'administration de plasmides codant pour env de

FIV un phénomène de facilitation. Ce phénomène a aussi été observé lors de vaccination à l'aide de protéines recombinées de FIV (Lutz H. et al., AIDS Research and Human Retroviruses, 1996, 12(5), 431-433) et de virus de la vaccine exprimant env de FIV (Osterhaus A. D. M. E. et al., AIDS Research and Human Retroviruses, 1996, 12(5), 437-441).

WO-A-98/21354 décrit un protocole de vaccination contre FIV dans lequel on administre d'abord un vecteur canarypox recombiné ayant comme insert env et gag/pro de FIV, puis du FIV inactivé produit sur lymphocytes T.

Dans Cuisinier A. M. *et. al*., Vaccine, 1999, **17**, 415-425, l'administration d'ADN exprimant gp120 est là encore à l'origine de phénomènes de facilitation.

La demande de brevet WO-A-9530019 décrit un protocole de vaccination contre FIV utilisant l'expression de diverses protéines de FIV par des vecteurs de type Feline herpesvirus ou baculovirus pour une stratégie primoadministration / rappel.

La demande de brevet EP-A-1074625 décrit un protocole de vaccination polynucléotidique des chats contre FIV. La stratégie utilise aussi bien en primoadministration qu'en rappel des plasmides exprimant notamment protéines env et gag-pro de FIV en intramusculaire chez le chat à 4 semaines d'intervalle.

L'article de I. RAMSHAW ET AL, "The prime-boost strategy: exciting prospects for improved vaccination" IMMUNOLOGY TODAY, vol. 21, no. 4, 2000, pages 163-165, passe en revue les essais de stratégie d'immunisation par primoadministration avec un ADN codant pour les protéines virales et rappels avec des vecteurs poxviraux dans le cas d'infection lentivirale. Rawshaw et al. soulignent que les résultats varient mais que la séquence ADN en primovaccination/ vecteur poxviral en rappel, ainsi que le choix du vecteur poxviral s'avèrent cruciaux pour une bonne réponse immunitaire.

Différentes approches de vaccination ont donc été étudiées à savoir les vaccins Inactivés, les vaccins de sous-unités et les vaccins recombinés (vecteurs viraux et ADN). Il n'existe pas à ce jour de solution satisfaisante, ni d'orientation claire de recherche.

Après des efforts de recherches importants, la déposante a pu mettre au point une technique d'induction d'une réponse immunitaire chez le chat contre FIV utilisant des compositions immunogènes recombinées de type vecteur viral et ADN (plasmide). De façon remarquable, la technique mise au point permet d'utiliser l'expression d'env sans générer de problème de facilitation.

Ainsi, l'invention est définie par les revendications et a pour premier objet une méthode d'induction d'une réponse immunitaire chez le chat contre FIV par un protocole d'administration comprenant au moins une prime-administration et au moins une administration de rappel mettant en oeuvre au moins les immunogènes communs gag/pro et env. Les compositions immunogènes utilisées en primo-administration sont de nature différente de celles utilisées en rappel. Ce protocole d'administration est dit "prime-boost". Le protocole prime-boost selon l'invention comprend une primo-administration intramusculaire avec une composition immunogène comprenant, dans un véhicule ou excipient pharmaceutiquement acceptable, un plasmide contenant et exprimant *in vivo* un polynucléotide codant pour env et gag/pro de FIV, suivie d'un rappel à un intervalle de 3 à 6 semaines avec une composition immunogène comprenant, dans un véhicule ou excipient pharmaceutiquement acceptable, un vecteur viral atténué contenant et exprimant *in vivo* un polynucléotide codant pour env et gag/pro de FIV, le vecteur viral étant un virus canarypox ou fowlpox atténué.

La primo-administration peut comprendre une ou plusieurs administrations des mêmes compositions immunogènes à base de plasmides. De même l'administration de rappel peut comprendre une ou plusieurs administrations des mêmes compositions immunogènes à base de vecteurs avipoxviraux atténués. Suivant un mode de réalisation particulier de l'invention, le protocole comprend deux administrations successives d'une même composition immunogène à base de plasmides, puis une administration d'une composition immunogène à base de vecteurs avipoxviraux atténués à titre de rappel.

Les différentes administrations sont faites à un intervalle de 3 à 6 semaines et plus particulièrement de l'ordre de 4 semaines. Suivant une modalité préférée on prévoit en plus un rappel annuel, préférentiellement à l'aide de la composition immunogène à base de vecteurs viraux. De préférence les animaux sont âgés d'au moins 6 à 8 semaines lors de la première administration.

Les plasmides et les vecteurs viraux utilisés dans le protocole prime/boost expriment à la fois env et gag/pro.

Au sens de l'invention, le terme protéine englobe les fragments, y compris peptides et polypeptides possédant substantiellement la même activité immunologique que la protéine entière. Les expressions gène env, gène gag, gène gag/pro, etc. incluent donc les fragments polynucléotidiques codant pour ces fragments de protéine, peptides et polypeptides.

Pour contrôler leur expression, les polynucléotides sont fonctionnellement associés à un promoteur, et éventuellement à des séquences activatrices (en anglais « enhancer »), à des séquences stabilisatrices, et/ou à des séquences signal permettant la sécrétion de la protéine.

D'autres protéines de FIV peuvent être exprimées conjointement à env et gag/pro, en primo-administration et/ou en rappel. On peut notamment exprimer la protéine tat et/ou la protéine rev, de préférence tat et optionnellement rev. Elles peuvent être exprimées dans les mêmes plasmides et/ou vecteurs viraux que ceux utilisés pour env et gag/pro ou dans des plasmides et/ou vecteurs viraux propres.

Suivant une modalité particulière, plusieurs souches de FIV sont représentées dans les compositions de l'invention, c'est-à-dire que le protocole comprend l'administration de plasmides et de vecteurs viraux comportant et exprimant *in vivo* des polynucléotides codant pour env et gag/pro de deux, trois ou plus, souches de FIV. Ainsi, des polynucléotides de plusieurs souches de FIV peuvent être portés par un vecteur viral unique ou par des vecteurs viraux propres. Pour les plasmides, on préfère des plasmides propres, mais on n'exclut cependant pas qu'un plasmide unique comporte les polynucléotides de plusieurs souches de FIV.

Suivant une autre modalité encore, et comme on le verra plus en détail plus loin, le protocole peut comprendre l'administration concomitante d'une ou plusieurs cytokines, soit sous forme protéique, soit par incorporation d'un polynucléotide codant pour une cytokine dans des plasmides et/ou des vecteurs viraux, ceux utilisés pour exprimer les protéines FIV et/ou d'autres.

Pour réaliser les vecteurs d'expression selon l'invention l'homme du métier dispose de différentes souches de FIV, de l'organisation de leur génome et de la séquence nucléotidique de leur génome. Des souches FIV utilisables, telle que la souche Petaluma, sont citées dans la partie Exemples. Des informations complémentaires sur ces souches ainsi que d'autres, leurs séquences nucléotidiques, sont données en introduction de la partie Exemples.

Selon l'invention, la primo-administration comprend l'utilisation de plasmides exprimant *in vivo* les protéines env et gag de FIV. Le terme plasmide se réfère à une unité de transcription ADN comprenant un polynucléotide selon l'invention et les éléments nécessaires à son expression *in vivo.* On préfère la forme plasmide circulaire, superenroulée ou non. La forme linéaire entre également dans le cadre de cette invention.

Chaque plasmide comprend un promoteur apte à assurer, dans les cellules hôtes, l'expression du polynucléotide inséré sous sa dépendance. Il s'agit en général d'un promoteur eucaryote fort. Le promoteur eucaryote fort préféré est le promoteur précoce du cytomégalovirus (CMV-IE), d'origine humaine ou murine, ou encore éventuellement d'une autre origine telle que rat, cobaye. Le promoteur CMV-IE peut comprendre la partie promoteur proprement dite, associée ou non à la partie activatrice (enhancer). On peut se référer à EP-A-260 148, EP-A-323 597, US-A-5 168 062, US-A-5 385 839, US-A-4 968 615, WO-A-87/03905. On préfère le CMV-IE humain (Boshart M. et al., Cell., 1985, 41, 521-530) ou murin.

De manière plus générale, le promoteur est soit d'origine virale, soit d'origine cellulaire. Comme promoteur viral fort autre que CMV-IE, on peut citer le promoteur précoce ou le promoteur tardif du virus SV40 ou le promoteur LTR du virus du Sarcome de Rous. Comme promoteur cellulaire fort, on peut citer le promoteur d'un gène du cytosquelette, tel que par exemple le promoteur de la et al., Vaccine, 2000, 18(22), 2337-2344), ou encore le promoteur de l'actine (Miyazaki J. et al., Gene, 1989, 79(2), 269-277).

Par équivalence, les sous-fragments de ces promoteursn conservant une activité promotrice adéquate sont compris dans la présente invention: e.g. les promoteurs CMV-IE tronqués selon WO-A-98/00166. La notion de promoteur selon l'invention inclut donc les dérivés et sous-fragments conservant une activité promotrice adéquate, de préférence substantiellement similaire à celle du promoteur proprement dit dont ils sont issus. Pour le CMV-IE, cette notion comprend la partie promoteur proprement dite et/ou la partie activatrice et les dérivés et sous-fragments.

De préférence les plasmides comprennent d'autres éléments de contrôle de l'expression. En particulier, il est avantageux d'incorporer des séquences stabillsatrices du type intron, de préférence intron Il du gène de de la B-globine de lapin (van Ooyen et al. Science, 1979, 206:337-344).

Comme signal de polyadénylation (polyA) pour les plasmides on peut utiliser notamment celui du gène de l'hormone de croissance bovine (bGH) (US-A-5,122,458), celui du gène de la β-globine du lapin ou celui du virus SV40.

Selon l'invention, l'administration de rappel comprend l'utilisation de vecteurs viraux exprimant in *in vivo* la ou les protéines de FIV. . Ces vescteurs d'expressuion viraux sont des canarypox ou des fowlpox.

Pour le fowlpox, l'homme du métier peut se reporter à US-A-5, 174 US-5,766,599; pour le canarypox à US-A-5,756,103.

Selon l'un des modes préférés de l'invention, le vecteur d'expression poxvirus est un virus canarypox_{,} atténué, e.g. un ALVAC ou un virus canarypox (par exemple de la souche Rentschler) qui a été atténué, notamment par plus de 200 passages sur cellules de fibroblastes d'embryons de poulet (CEF). Un virus canarypox de souche ALVAC a été déposé le 14 novembre 1996 auprès de l'American Type Culture Collection (ATCC) sous le numéro d'accès VR-2547. Un canarypox est commercialement disponible auprès de l'ATCC sous le numéro d'accès VR-111. Des canarypox atténués ont été décrits dans US-A-5,756,103 et dans WO-A-01/05934.

Des fowlpox atténués peuvent être utilisés (e.g. TROVAC). Sur le poxvirus TROVAC, l'homme de l'art petit se reporter au brevet WO-A-96/40241. De nombreuses souches vaccinales de virus fowlpox sont accessibles, par example le vaccin DIFTOSEC CT® commercialisé par MERIAL et le vaccin NOBILIS® VARIOLE commercialisé par Intervet.

Lorsqu'il s'agit d'un canarypox, les sites d'insertion sont en particulier situés dans, ou constitués par, les COLs C3, C5 et C6. Lorsqu'il s'agit d'un fowlpox, les sites d'insertion sont en particulier situés dans, ou constitués par, les COLs F7 et F8.

De préférence, lorsque le vecteur d'expression est un poxvirus, le polynucléotide à exprimer est inséré sous le contrôle d'un promoteur spécifique des poxvirus, notamment le promoteur vaccine 7,5 kDa (Cochran et al., J. Virology, 1985, 54, 30-35), le promoteur vaccine I3L (Riviere et al., J. Virology, 1992, 66, 3424-3434), le promoteur vaccine HA (Shida, Virology, 1986, 150, 451-457), le promoteur cowpox ATI (Funahashi et al., J. Gen. Virol., 1988, 69, 35-47), ou encore le promoteur vaccine H6 (Taylor J. et a/. Vaccine, 1988, 6, 504-508 ; Guo P. et al. J. Virol., 1989, 63, 4189-4198 ; Perkus M. et al. J. Virol., 1989, 63, 3829-3836).

L'invention a également pour objet l'utilisation d'une part de plasmides contenant et exprimant *in vivo* le/les polynucléotide(s) codant pour env et gag/pro de FIV, pour la production d'une première préparation immunogène comprenant le où les plasmide(s) et un véhicule ou excipient pharmaceutiquement acceptable, pour une primo-administration à un chat, et d'autre part un ou des vecteur(s) viral(aux) atténué(s) exprimant *in vivo* env et gag/pro de FIV, pour la production d'une seconde composition immunogène comprenant le ou les vecteur(s) viral(aux) atténué(s) et un véhicule ou excipient pharmaceutiquement acceptable, pour une administration en rappel à un intervalle de 3 à 6 semaines au même chat,
dans laquelle
l'administration de la première et la seconde composition immunogène se fait par voie intramusculaire, et
le ou les vecteur(s) viral(aux) atténué(s) sont un ou des virus canarypox atténué(s) ou un ou des virus fowlpox atténué(s).

L'invention a également pour objet l'utilisation d'un ou de plusieurs plasmide(s) exprimant *in vivo* env et gag/pro de FIV, pour la production d'une composition immunogène comprenant le ou les plasmide(s) et un véhicule ou excipient pharmaceutiquement acceptable pour l'induction d'une réponse immunitaire dirigée contre FIV chez un chat, destiné à être administré par voie intramusculaire au chat en primo-administration, le rappel étant effectué après 3 à 6 semaines à l'aide d'une composition immunogène comprenant un ou plusieurs vecteur(s) viral(aux) atténué(s) exprimant *in vivo* env et gag/pro de FIV et un véhicule ou excipient pharmaceutiquement acceptable,
dans laquelle le ou les vecteur(s) viral(aux) atténué(s) sont un ou des virus canarypox atténué(s) ou un ou des virus fowlpox atténué(s).

L'invention a également pour objet l'utilisation d'un ou plusieurs vecteur(s) viral(aux) atténué(s)exprimant *in vivo* env et gag/pro de FIV, pour la production d'une composition immunogène comprenant le ou les vecteur(s) viral(aux) atténué(s) et un véhicule ou excipient pharmaceutiquement acceptable, pour l'induction d'une réponse immunitaire dirigée contre FIV chez un chat, destiné à être administré par voie intramusculaire au chat en rappel à un intervalle de 3 à 6 semaines d'une composition immunogène comprenant un ou plusieurs plasmide(s) exprimant *in vivo* env et gag/pro de FIV et un véhicule ou excipient pharmaceutiquement acceptable,
dans laquelle le ou les vecteur(s) viral(aux) atténué(s) sont un ou des virus canarypox atténué(s) ou un virus fowlpox atténué(s).

La présente invention a encore pour objet un kit pour l'induction d'une réponse immunitaire dirigée contre FIV chez un chat notamment destiné à être utilisé selon le protocole d'administration selon l'Invention, comprenant, conditionnés séparément :
- une première composition immunogène comprenant, dans un véhicule ou excipient pharmaceutiquement acceptable, un ou des plasmide(s) exprimant *in vivo* env et gag/pro de FIV,
- une seconde composition immunogène comprenant, dans un véhicule ou excipient pharmaceutiquement acceptable, un ou des vecteur(s) viral(aux) atténué(s) exprimant *in vivo* env et gag/pro de FIV, dans laquelle le ou les vecteur(s) viral(aux) atténué(s) sont un ou des virus canarypox atténué(s) ou un ou des virus fowlpox atténué(s).

Il va de soi que l'ensemble des caractéristiques décrites dans la présente demande, qui concernent par exemple la composition des plasmides et vecteurs viraux, la composition des préparations, les associations de polynucléotides de FIV, le protocole d'administration s'appliquent dans les mêmes conditions aux différents objets de l'invention.

La notion de composition immunogène recouvre toute composition susceptible, une fois administrée à l'espèce cible dans les conditions de l'invention, d'induire une réponse Immunitaire dirigée contre FIV. L'espèce cible est le chat.

Les véhicules ou excipients pharmaceutiquement acceptables sont parfaitement connus de l'homme du métier. A titre d'exemple, il peut s'agir de solution saline NaCl à 0,9% ou de tampon phosphate. Les véhicules ou excipients pharmaceutiquement acceptables englobent aussi tout composé ou combinaison de composés permettant la facilitation de l'administration du vecteur, notamment de la transfection, et/ou l'amélioration de la conservation.

Les compositions immunogènes selon l'invention comprennent de préférence un ou plusieurs adjuvants, choisis notamment parmi les adjuvants usuels. Conviennent particulièrement bien dans le cadre de la présente invention : (1) polymères de l'acide acrylique ou méthacrylique, polymères d'anhydride maléique et de dérivé alcényle, (2) séquences immunostimulatrices (ISS), notamment séquences oligodésoxyribonucléotidiques ayant un ou plusieurs motifs CpG non méthylés (Klinman D. M. et al., Prcc. Natl. Acad. Sci. USA, 1996, 93, 2879-2883 ; WO-A1-98/16247), (3) une émulsion huile-dans-l'eau, en particulier l'émulsion SPT décrite à la page 147 de « Vaccine Design, The Subunit and Adjuvant Approach » edited by M. Powell, M. Newman, Plenum Press 1995, et l'émulsion MF59 décrite à la page 183 du même ouvrage, (4) lipides cationiques contenant un sel d'ammonium quaternaire, (5) cytokines, ou (6) leurs combinaisons ou mélanges.

L'émulsion huile-dans-l'eau (3), qui est particulièrement adaptée pour les vecteurs viraux, peut notamment être à base :
- d'huile de paraffine liquide légère (type Pharmacopée européenne) ;
- d'huile isoprénoide telle que le squalane, le squalène ;
- d'huile résultant de l'oligomérisation d'alcènes, en particulier d'isobutène ou de decène ;
- d'esters d'acides ou d'alcools à groupement alkyle linéaire ;
- plus particulièrement huiles végétales, oléate d'éthyle, di(caprylate / caprate) de propylène glycol, tri(caprylate / caprate) de glycérol, dioléate de propylène glycol ;
- d'esters d'acides ou d'alcools gras ramifiés, en particulier esters de l'acide isostéarique.

L'huile est utilisée en association avec des émulsifiants pour former l'émulsion. Les émulsifiants sont de préférence des tensio-actifs non ioniques, en particulier :
- les esters d'une part de sorbitan, de mannide (e.g. oléate d'anhydromannitol), de glycérol, de polyglycérol, ou de propylène glycol et d'autre part d'acide oléique, isostéarique, ricinoléique, hydroxystéarique, ces esters étant éventuellement éthoxylés,
- les blocs copolymères polyoxypropylène-polyoxyéthylène, en particulier les Pluronic®, notamment L121.

Parmi les polymères adjuvants de type (1), on préfère les polymères de l'acide acrylique ou méthacrylique réticulés, notamment réticulés par des éthers polyalcényliques de sucres ou de polyalcools. Ces composés sont connus sous le terme carbomère (Pharmeuropa vol. 8, n° 2, juin 1996). L'homme de l'art peut aussi se référer à US-A-2 909 462 qui décrit de tels polymères acryliques réticulés par un composé polyhydroxylé ayant au moins 3 groupes hydroxyle, de préférence pas plus de 8, les atomes d'hydrogène d'au moins trois hydroxyles étant remplacés par des radicaux aliphatiques insaturés ayant au moins 2 atomes de carbone. Les radicaux préférés sont ceux contenant de 2 à 4 atomes de carbone, e.g. vinyles, allyles et autres groupes éthyléniquement insaturés. Les radicaux insaturés peuvent eux-mêmes contenir d'autres substituants, tels que méthyl. Les produits vendus sous la dénomination Carbopol® (BF Goodrich, Ohio, USA) sont particulièrement appropriés. Ils sont notamment réticulés par un allyl saccharose ou par de l'allylpentaérythritol. Parmi eux, on peut citer en particulier les Carbopol® 974P, 934P et 971 P.

La concentration en polymère de type carbomère dans la composition vaccinale finale peut notamment aller de 0,01 % à 1,5 % PN, plus particulièrement de 0,05 à 1 % P/V, de préférence de 0,1 à 0,4 % PN.

Les lipides cationiques (4) contenant un sel d'ammonium quaternaire, qui sont particulièrement mais pas exclusivement adaptés pour les plasmides, sont de préférence ceux qui répondent à la formule suivante : 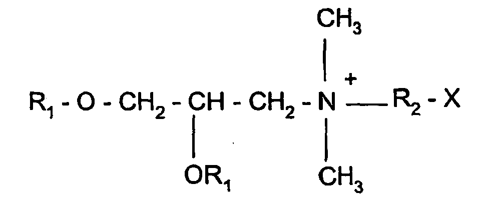 dans laquelle R1 est un radical aliphatique linéaire, saturé ou insaturé, ayant 12 à 18 atomes de carbone, R2 est un autre radical aliphatique, renfermant 2 ou 3 atomes de carbone, et X un groupement hydroxyle ou amine.

Parmi ces lipides cationiques, on préfère le DMRIE (N-(2-hydroxyéthy)-N,N-diméthyl-2,3-bis(tetradécyloxy)-1-propanammonium ; WO-A-96/34109), de préférence associé avec un lipide neutre, de préférence le DOPE (dioléoyl-phosphatidyl-éthanolamine ; Behr J. P., 1994, Bioconjugate Chemistry, 5, 382-389), pour former le DMRIE-DOPE.

De préférence, le mélange plasmide avec cet adjuvant se fait de manière extemporanée et l'on préfère, avant son administration, laisser le temps au mélange ainsi constitué de se complexer, par exemple pendant une durée allant de 10 à 60 minutes, notamment de l'ordre de 30 minutes.

Lorsque du DOPE est présent, le ratio molaire DMRIE : DOPE va de préférence de 95 : 5 à 5 : 95, plus particulièrement de 1 : 1.

Le ratio pondéral plasmide : adjuvant DMRIE ou DMRIE-DOPE peut aller notamment de 50 : 1 à 1 : 10, en particulier de 10 : 1 à 1 : 5, et de préférence de 1 : 1 à 1 : 2.

La ou les cytokines (5) éventuellement présentes peuvent être apportées sous forme de protéine à la composition , ou être co-exprimées chez l'hôte avec le ou les protéines de FIV. La préférence va à la co-expression de la ou des cytokines, soit par le même vecteur que celui exprimant les protéines, soit par un vecteur propre.

Les cytokines peuvent notamment être choisies parmi des cytokines félines, notamment celles du chat, telles que l'interleukine 18 féline (fIL-18) (Taylor S. et al., DNA Seq., 2000, 10(6), 387-394), fIL-16 (Leutenegger C. M. et al., DNA Seq., 1998, 9(1), 59-63), flL-12 (Fehr D. et al., DNA Seq., 1997, 8(1-2), 77-82 ; Imamura T. et al., J. Vet. Med. Sci., 2000, 62(10), 1079-1087) et GM-CSF félin (facteur de stimulation de la formation de colonies granulo-macrophagiques, ou en anglais Granulocyte-Macrophage Colony-Stimulating Factor) (GenBank AF053007).

Conformément à l'invention l'induction d'une reponse immunitaire dirigée contre FIV peut être associée à des vaccinations contre d'autres agents pathogènes félins. Les autres pathogènes félins sont notamment le virus de la rhinotrachéite féline ou virus herpès félin (FHV), les virus de la leucémie féline (FeLV de type A et de type B), les parvovirus félins (FPV), le virus de la péritonite infectieuse féline (FIPV), le virus de la calicivirose féline (FCV), le virus de la rage, *Chlamydia.*

L'administration des compositions selon l'invention se fait par la voie , intramusculaire,

Les différentes compositions peuvent être injectées par un injecteur à jet liquide sans aiguille.

Les compositions immunogènes selon l'invention comprennent une quantité efficace de plasmide ou de vecteur viral, la détermination de ces quantités étant à la portée de l'homme du métier. La déposante recommande :
- dans le cas des compositions immunogènes à base de plasmide, une dose peut comporter de 1 µg environ à 2000 µg environ, notamment de 50 µg environ à 1000 µg environ. Les volumes de dose peuvent être compris entre 0,1 et 2 ml, de préférence entre 0,2 et 1 ml.
- dans le cas des compositions immunogènes à base de poxvirus, une dose peut être comprise entre environ 10³ pfu et environ 10⁹ pfu. Lorsque le vecteur est le virus canarypox, la dose est plus particulièrement comprise entre environ 10⁵ pfu et environ 10⁹ pfu, de préférence entre environ 10⁶ et environ 10⁸ pfu. Les volumes de dose des compositions immunogènes à base de vecteurs viraux sont en général compris entre 0,1 et 2,0 ml, de préférence entre 0,2 et 1,0 ml.

Le kit peut comprendre les doses de composition immunogène pour induires une réponse immunitaire soit chez un animal, soit chez plusieurs animaux.

Selon une modalité particulière, le kit comprend deux doses de composition immunogène à base de plasmide pour une dose de composition immunogène à base de vecteur viral.

L'invention va être maintenant décrite plus en détail à l'aide de modes de réalisation pris à titre d'exemples non limitatifs.

### Exemples :

Toutes les constructions sont réalisées en utilisant les techniques standards de biologie moléculaire (clonage, digestion par les enzymes de restriction, synthèse d'un ADN complémentaire simple brin, amplification en chaîne par polymérase, élongation d'un oligonucléotide par une ADN polymérase...) décrites par Sambrook J. et al. (Molecular Cloning: A Laboratory Manual. 2nd Edition. Cold Spring Harbor Laboratory. Cold Spring Harbor. New York. 1989). Tous les fragments de restriction utilisés pour la présente invention, ainsi que les divers fragments d'amplification en chaîne par polymérase (PCR), sont isolés et purifiés en utilisant le kit "Geneclean®" (BIO101 Inc. La Jolla, CA).

Les exemples mettent en oeuvre la souche FIV Villefranche IFFA 1/88 (Steffan A. M. et al., J. Gen. Virol., 1994, 75, 3647-3653). Il va de soi que l'invention peut être appliquée aux autres souches de FIV. On peut par exemple citer la souche Petaluma (disponible auprès de l'American Type Culture Collection (ATCC) sous le numéro VR-1312, et de la séquence nucléotidique dans GenBank sous le numéro M25381 ; gène env: nucléotides 6266 à 8836 ; gène gag : nucléotides 628 à 1980 ; gag/pro : nucléotides 628 à 2336). On peut citer aussi la souche NCSU1 disponible auprès de l'ATCC sous la référence VR2333. On peut se référer aussi aux articles de Sodora et de Bachmann (Sodora D. L. et al., J. Virol., 1994, 68(4), 2230-2238 ; Bachmann M. H. et al., J. Viral., 1997, 71(6), 4241-4253) qui décrivent un certain nombre de souches FIV et indiquent les références d'accès aux séquences dans GenBank. La souche FIV14 est référencée NC_001482 dans Genbank (gène env : nucléotides 6266-8836 ; gène gag : nucléotides 628-1980 ; gag/pro: nucléotides 628-2336), la souche BM3070 est référencée dans GenBank sous AF474246 (gène env : nucléotides 6272-8833 ; gène gag : nucléotides 634-1986) ; la souche OMA est référencée dans Genbank sous U56928 (gène env : nucléotides 6506-9097 ; gène gag : nucléotides 679-2179), etc.

L'homme du métier est capable de déterminer les sondes PCR pour cloner les gènes à partir de la souche FIV considérée. Les sondes PCR utilisées dans les exemples suivants pour cloner env, gag/pro, rev et tat de la souche Villefranche, peuvent être utilisées avec d'autres souches, telles que Petaluma ; ou être adaptées lorsque cela est utile.

### Exemple 1 : Culture du virus FIV

Pour leur amplification, des virus de l'immunodéficience féline de souche Villefranche IFFA 1/88 sont cultivées sur cellules Q201 (lymphocytes T auxiliaires félins ; Willet B. et al., J. Gen. Virol., 1997, 78, 611-618).

Les cellules Q201 sont mises en culture en Falcon 25 cm² avec du milieu Eagle-MEM supplémenté de 2 mM de glutamine, de 10 % de sérum de veau, de 100 UI/ml de pénicilline, de 100 µg/ml de streptomycine et de 100 UI/ml d'interleukine-2 humaine recombinée, contenant environ 100 000 cellules par ml. Les cellules sont cultivées à +37°C.

Après 3 jours la couche cellulaire arrive à confluence. Le milieu de culture est alors remplacé et le virus FIV est ajouté à raison de 5 pfu/cellule.

Lorsque l'effet cytopathogène (CPE) est complet (généralement 48-72 heures après le début de la mise en culture), les suspensions virales sont récoltées, puis clarifiées par centrifugation et congelées à -80°C. 3 à 4 passages successifs sont généralement nécessaires à la production d'un lot viral. Le lot viral est stocké à -80°C.

### Exemple 2 : Extraction de l'ARN viral de FIV

L'ARN viral contenu dans 100 ml de suspension virale de la souche FIV Villefranche est extrait après décongélation avec les solutions du kit « High Pure^{™} Viral RNA Kit » Cat # 1 858 882, Roche Molecular Biochemicals), en suivant les instructions du fournisseur pour les étapes d'extraction. Le culot d'ARN obtenu à la fin de l'extraction est resuspendu avec 1 à 2 ml d'eau distillée stérile sans RNase.

### Exemple 3 : Construction du plasmide pPB371

L'ADN complémentaire (ADNc) de FIV est synthétisé avec le kit « Gene Amp RNA PCR Kit » (Cat # N 808 0017, Perkin-Elmer, Norwalk, CT 06859, USA) en utilisant les conditions données par le fournisseur.

Une réaction de transcription inverse, suivie d'une réaction d'amplification en chaîne (Réaction « RT-PCR ») est réalisée avec 50 µl de la suspension d'ARN viral de FIV (exemple 2) et avec les oligonucléotides suivants :
FC116 (36 mer) (SEQ ID NO :1)
5' TTTTTTCTGCAGCAATAAGAATGGCAGAAGGATTTG 3'
et FC117 (36 mer) (SEQ ID NO :2)
5'-TCGCACCTGAAACATCTCGAGTGTTTCCACATGTAT 3'.

Ce couple d'oligonucléotides permet l'incorporation d'un site de restriction Pstl, d'un site de restriction Xhol et d'un codon ATG initiateur en 5' de l'insert.

La synthèse du premier brin d'ADNc se fait par élongation de l'oligonucléotide FC117, après hybridation de ce dernier à la matrice d'ARN.

Les conditions de synthèse du premier brin d'ADNc sont une température de 42°C pendant 15 min, puis de 99°C pendant 5 min, et enfin de 4°C pendant 5 min. Les conditions de la réaction PCR en présence du couple d'oligonucléotides FC116 et FC117 sont une température de 95°C pendant 2 min, puis 40 cycles (95°C pendant 30 sec, puis 50°C pendant 45 sec, et 72°C pendant 3 min), et enfin 72°C pendant 7 min pour produire un fragment de 1476 pb.

Ce fragment est digéré par l'enzyme de restriction Pstl puis par l'enzyme de restriction Xhol pour isoler, après électrophorèse en gel d'agarose, le fragment Pstl-Xhol d'environ 1450 pb. Ce fragment est appelé fragment A.

Une deuxième réaction de transcription inverse, suivie d'une réaction d'amplification en chaîne (Réaction « RT-PCR ») est réalisée avec 50 µl de la suspension d'ARN viral de FIV (exemple 2) et avec les oligonucléotides suivants :
FC118 (36 mer) (SEQ ID NO :3)
5' ATACATGTGGAAACACTCGAGATGTTTCAGGTGCGA 3'
et FC119 (54 mer) (SEQ ID NO :4)
5'TTTTTTGGATCCCCCGGGCTGCAGGAATTCTGAGATACTTCATCATTCC TCCTC 3'.

Ce couple d'oligonucléotides permet l'incorporation d'un site de restriction Xhol, d'un site de restriction BamHI et d'un codon stop en 3' de l'insert.

La synthèse du premier brin d'ADNc se fait par élongation de l'oligonucléotide FC118, après hybridation de ce dernier à la matrice d'ARN.

Les conditions de synthèse du premier brin d'ADNc sont une température de 42°C pendant 15 min, puis de 99°C pendant 5 min, et enfin de 4°C pendant 5 min. Les conditions de la réaction PCR en présence du couple d'oligonucléotides FC118 et FC119 sont une température de 95°C pendant 2 min, puis 40 cycles (95°C pendant 130 sec, puis 50°C pendant 45 sec, et 72°C pendant 3 min), et enfin 72°C pendant 7 min pour produire un fragment de 1193 pb.

Ce fragment est digéré par l'enzyme de restriction Xhol puis par l'enzyme de restriction BamHI pour isoler, après électrophorèse en gel d'agarose, le fragment XhoI-BamHI d'environ 1170 pb. Ce fragment est appelé fragment B.

Les fragments A et B sont ligaturés avec le plasmide d'expression eucaryote pVR1012 (figure 1 et exemple 7 de WO-A-98/03199 ; Hartikka J. et al., 1997, Human Gene Therapy, 7, 1205-1217) préalablement digéré par Xbal et EcoRI, pour donner le plasmide pPB371 (7467 pb). Ce plasmide contient, sous le contrôle du promoteur précoce du cytomégalovirus humain ou hCMV-IE (human Cytomegalovirus Immediate Early), un insert codant pour la protéine env de FIV.

### Exemple 4 : Construction du plasmide pPB374

L'ADN complémentaire (ADNc) de FIV est synthétisé avec le kit « Gene Amp RNA PCR Kit » (Cat # N 808 0017, Perkin-Elmer, Norwalk, CT 06859, USA) en utilisant les conditions données par le fournisseur.

Une réaction de transcription inverse, suivie d'une réaction d'amplification en chaîne (Réaction « RT-PCR ») est réalisée avec 50 µl de la suspension d'ARN viral de FIV (exemple 2) et avec les oligonucléotides suivants :
PB670 (37 mer) (SEQ ID NO :5)
5' TTTGTCGACAAGGTAGGAGAGATTCTACAGCAACATG 3'
et PB674 (40 mer) (SEQ ID NO :6)
5' TTTGCGGCCGCGTTATTGAGCCATTACTAACCTAATATTG 3'.

Ce couple d'oligonucléotides permet l'incorporation d'un site de restriction Sall, d'un site de restriction Notl, d'un codon ATG initiateur en 5' et d'un codon stop en 3' de l'insert.

La synthèse du premier brin d'ADNc se fait par élongation de l'oligonucléotide PB674, après hybridation de ce dernier à la matrice d'ARN.

Les conditions de synthèse du premier brin d'ADNc sont une température de 42°C pendant 15 min, puis de 99°C pendant 5 min, et enfin de 4°C pendant 5 min. Les conditions de la réaction PCR en présence du couple d'oligonucléotides PB670 et PB674 sont une température de 95°C pendant 2 min, puis 40 cycles (95°C pendant 30 sec, puis 50°C pendant 45 sec, et 72°C pendant 3 min), et enfin 72°C pendant 7 min pour produire un fragment de 1758 pb.

Ce fragment est digéré par l'enzyme de restriction Sall puis par l'enzyme de restriction Notl pour isoler, après électrophorèse en gel d'agarose, le fragment Sall-Notl d'environ 1750 pb. Ce fragment est ligaturé avec le plasmide d'expression pVR1012 (exemple 3) préalablement digéré par Sall et Notl, pour donner le plasmide pPB374 (6633 pb). Ce plasmide contient, sous le contrôle du promoteur précoce hCMV-IE un insert codant les protéines gag/pro de FIV.

### Exemple 5 : Construction du plasmide pPB375

L'ADN complémentaire (ADNc) de FIV est synthétisé avec le kit « Gene Amp RNA PCR Kit » (Cat # N 808 0017, Perkin-Elmer, Norwalk, CT 06859, USA) en utilisant les conditions données par le fournisseur.

Une réaction de transcription inverse, suivie d'une réaction d'amplification en chaîne (Réaction « RT-PCR ») est réalisée avec 50 µl de la suspension d'ARN viral de FIV (exemple 2) et avec les oligonucléotides suivants :
FC116 (36 mer) (SEQ ID NO :1)
et FC120 (48 mer) (SEQ ID NO :7)
5' TTTTTACCTGCATTTCCTTCTTCCAGTTTTACCTCTTGAATTTCGTTC 3'.

Ce couple d'oligonucléotides permet l'incorporation d'un site de restriction Pstl, d'un site de restriction BspMI et d'un codon ATG initiateur en 5' de l'insert.

La synthèse du premier brin d'ADNc se fait par élongation de l'oligonucléotide FC120, après hybridation de ce dernier à la matrice d'ARN.

Les conditions de synthèse du premier brin d'ADNc sont une température de 42°C pendant 15 min, puis de 99°C pendant 5 min, et enfin de 4°C pendant 5 min. Les conditions de la réaction PCR en présence du couple d'oligonucléotides FC116 et FC120 sont une température de 95°C pendant 2 min, puis 40 cycles (95°C pendant 30 sec, puis 50°C pendant 45 sec, et 72°C pendant 3 min), et enfin 72°C pendant 7 min pour produire un fragment de 265 pb.

Ce fragment est digéré par l'enzyme de restriction PstI puis par l'enzyme de restriction BspMI pour isoler, après électrophorèse en gel d'agarose, le fragment PstI-BspMI d'environ 240 pb. Ce fragment est nommé fragment C.

Une deuxième réaction de transcription inverse, suivie d'une réaction d'amplification en chaîne (Réaction « RT-PCR ») est réalisée avec 50 µl de la suspension d'ARN viral de FIV (exemple 2) et avec les oligonucléotides suivants :
PB672 (48 mer) (SEQ ID NO :8)
5' TTTACTGGAAGAAGGAAATGCAGGTAAAAGGAAAAGACAAAGAAGAAG 3'
et PB673 (36 mer) (SEQ ID NO :9)
5'TTTAGATCTTTAGTCCATAAGCATTCTTTCTATTTC 3'.

Ce couple d'oligonucléotides permet l'incorporation d'un site de restriction BspMI, d'un site de restriction BglII et d'un codon stop en 3' de l'insert.

La synthèse du premier brin d'ADNc se fait par élongation de l'oligonucléotide PB673, après hybridation de ce dernier à la matrice d'ARN.

Les conditions de synthèse du premier brin d'ADNc sont une température de 42°C pendant 15 min, puis de 99°C pendant 5 min, et enfin de 4°C pendant 5 min. Les conditions de la réaction PCR en présence du couple d'oligonucléotides PB672 et PB673 sont une température de 95°C pendant 2 min, puis 40 cycles (95°C pendant 30 sec, puis 50°C pendant 45 sec, et 72°C pendant 3 min), et enfin 72°C pendant 7 min pour produire un fragment de 246 pb.

Ce fragment est digéré par l'enzyme de restriction BspMI puis par l'enzyme de restriction BglII pour isoler, après électrophorèse en gel d'agarose, le fragment BspMI-BglII d'environ 230 pb. Ce fragment est nommé fragment D.

Les fragments C et D sont ligaturés avec le plasmide d'expression pVR1012 (exemple 3) préalablement digéré par les enzymes de restriction PstI et BglII, pour donner le plasmide pPB375 (5316 pb). Ce plasmide contient, sous le contrôle du promoteur précoce hCMV-IE un insert codant pour la protéine rev de FIV.

### Exemple 6 : Construction du plasmide pPB383

L'ADN complémentaire (ADNc) de FIV est synthétisé avec le kit « Gene Amp RNA PCR Kit » (Cat # N 808 0017, Perkin-Elmer, Norwalk, CT 06859, USA) en utilisant les conditions données par le fournisseur.

Une réaction de transcription inverse, suivie d'une réaction d'amplification en chaîne (Réaction « RT-PCR ») est réalisée avec 50 µl de la suspension d'ARN viral de FIV (exemple 2) et avec les oligonucléotides suivants :
PB680 (29 mer) (SEQ ID NO :10)
5' TTTCTGCAGATGGAAGACATAATAGTATT 3',
et PB681 (32 mer) (SEQ ID NO :11)
5' TTTAGATCTCTAAGCAGTAGTTATTGATAATG 3'.

Ce couple d'oligonucléotides permet l'incorporation d'un site de restriction BglII, d'un site de restriction Pstl, d'un codon ATG initiateur en 5' et d'un codon stop en 3' de l'insert.

La synthèse du premier brin d'ADNc se fait par élongation de l'oligonucléotide PB681, après hybridation de ce dernier à la matrice d'ARN.

Les conditions de synthèse du premier brin d'ADNc sont une température de 42°C pendant 15 min, puis de 99°C pendant 5 min, et enfin de 4°C pendant 5 min. Les conditions de la réaction PCR en présence du couple d'oligonucléotides PB680 et PB681 sont une température de 95°C pendant 2 min, puis 40 cycles (95°C pendant 30 sec, puis 50°C pendant 45 sec, et 72°C pendant 1 min), et enfin 72°C pendant 7 min pour produire un fragment de 254 pb.

Ce fragment est digéré par l'enzyme de restriction PstI puis par l'enzyme de restriction BglII pour isoler, après électrophorèse en gel d'agarose, le fragment Pstl-BglII d'environ 240 pb. Ce fragment (fragment E) est ligaturé avec le plasmide d'expression pVR1012 (exemple 3) préalablement digéré par PstI et BglII, pour donner le plasmide pPB383 (5089 pb). Ce plasmide contient, sous le contrôle du promoteur précoce hCMV-IE, un insert codant pour la protéine tat de FIV.

### Exemple 7: construction des virus recombinés vCP242, vCP253 et vCP255

Le brevet WO-A-98/21354 décrit en détail l'obtention des virus recombinés vCP242, vCP253 et vCP255, respectivement aux exemples 1, 2 et 4.

Le virus recombiné vCP242 comprend la séquence nucléotidique codant pour la protéine env de la souche Villefranche de FIV sous le contrôle d'un promoteur H6 du virus de la vaccine et insérée dans le site C6 de virus canarypox ALVAC.

Le virus recombiné vCP253 comprend la séquence nucléotidique codant pour les protéines gag/pro de la souche Villefranche de FIV sous le contrôle d'un promoteur I3L du virus de la vaccine et insérée dans le site C6 de virus canarypox ALVAC.

Le virus recombiné vCP255 comprend la séquence nucléotidique codant pour la protéine env de la souche Villefranche de FIV sous le contrôle d'un promoteur H6 du virus de la vaccine et la séquence nucléotidique codant pour les protéines gag/pro de la souche Villefranche de FIV sous le contrôle d'un promoteur I3L du virus de la vaccine, toutes deux insérées dans le site C6 de virus canarypox ALVAC.

### Exemple 8 : construction du plasmide donneur pour l'insertion dans le site C5 du virus canarypox ALVAC

La figure 16 du brevet US-A-5,756,103 montre la séquence d'un fragment de 3199 pb de l'ADN génomique du virus canarypox. L'analyse de cette séquence a révélé un cadre ouvert de lecture (COL) qui a été appelé C5L, qui commence à la position 1538 et se termine à la position 1859. La construction d'un plasmide d'insertion aboutissant à la délétion du COL C5L et à son remplacement par un site de clonage multiple flanqué de signaux d'arrêt de transcription et de traduction a été réalisée comme décrit ci-après.

Une réaction PCR a été réalisée à partir de la matrice constituée par l'ADN génomique du virus canarypox et avec les oligonucléotides suivants :
C5A1 (42 mer) (SEQ ID NO :12) :
5' ATCATCGAGCTCCAGCTGTAATTCATGGTCGAAAAGAAGTGC 3'
et FC121 (79 mer) (SEQ ID NO :13) :
5'GAATTCCTCGAGAGATCTCTGCAGCCCGGGTTTTTATAGCTAATTAGTC ATTTTTTGAGAGTACCACTTCAGCTACCTC 3'
pour isoler un fragment PCR de 229 pb (fragment B).

Une réaction PCR a été réalisée à partir de la matrice constituée par l'ADN génomique du virus canarypox et avec les oligonucléotides suivants :
FC122 (78 mer) (SEQ ID NO :14) :
5'CCCGGGCTGCAGAGATCTCTCGAGGAATTCTTTTTATTGATTAACTAGT CATTATAAAGATCTAAAATGCATAATTTC 3'
et C5D1 (45 mer) (SEQ ID NO :15) :
5' GATGATGGTACCGTAAACAAATATAATGAAAAGTATTCTAAACTA 3'
pour isoler un fragment PCR de 488 pb (fragment C).

Les fragments B et C ont été hybridés ensemble pour servir de matrice à une réaction PCR réalisée avec les oligonucléotides C5A1 (SEQ ID NO :12) et C5D1 (SEQ ID NO :15) pour générer un fragment PCR de 693 pb. Ce fragment a été digéré par les enzymes de restriction SacI et KpnI, pour isoler, après électrophorèse en gel d'agarose, un fragment SacI-KpnI de 676 pb. Ce fragment a été ligaturé avec le vecteur pBlueScript® II SK+ (Stratagene, La Jolla, CA, USA, Cat # 212205), préalablement digéré par les enzymes de restriction SacI et KpnI, pour donner le plasmide pFC115. La séquence de ce plasmide a été vérifiée par séquençage. Ce plasmide contient 166 pb de séquences situées en amont du COL C5L (« bras flanquant gauche C5 »), un signal vaccine d'arrêt précoce de transcription, des codons stops dans les 6 phases de lecture, un site de clonage multiple contenant les sites de restriction SmaI, PstI, BglII, XhoI et EcoRI, et enfin 425 pb de séquences situées en aval du COL C5L (« bras flanquant droit C5 »).

Le plasmide pMP528HRH (Perkus M. *et al*. J. Virol. 1989. **63**. 3829-3836) a été utilisé comme matrice pour amplifier la séquence complète du promoteur vaccine H6 (N° d'accès GenBank M28351) avec les oligonucléotides suivants :
JCA291 (34 mer) (SEQ ID NO :16)
5' AAACCCGGGTTCTTTATTCTATACTTAAAAAGTG 3'
et JCA292 (43 mer) (SEQ ID NO :17)
5' AAAAGAATTCGTCGACTACGATACAAACTTAACGGATATCGCG 3'
pour amplifier un fragment PCR de 149 pb. Ce fragment a été digéré par les enzymes de restriction Smal et EcoRI pour isoler, après électrophorèse en gel d'agarose, un fragment de restriction Smal-EcoRI de 138 pb. Ce fragment a alors été ligaturé avec le plasmide pFC115, préalablement digéré par Smal et EcoRI, pour donner le plasmide pFC116.

### Exemple 9: construction du plasmide donneur pour l'insertion dans le site C6 du virus canarypox ALVAC

La figure 4 du brevet WO-A-01/05934 montre la séquence d'un fragment de 3700 pb de l'ADN génomique du virus canarypox. L'analyse de cette séquence a révélé un cadre ouvert de lecture (COL) qui a été appelé C6L, qui commence à la position 377 et se termine à la position 2254. La construction d'un plasmide d'insertion aboutissant à la délétion du COL C6L et à son remplacement par un site de clonage multiple flanqué de signaux d'arrêt de transcription et de traduction a été réalisée comme décrit ci-après.

Une réaction PCR a été réalisée à partir de la matrice constituée par l'ADN génomique du virus canarypox et avec les oligonucléotides suivants :
C6A1 (42 mer) (SEQ ID NO :18) :
   5' ATCATCGAGCTCGCGGCCGCCTATCAAAAGTCTTAATGAGTT 3'
et FC123 (79 mer) (SEQ ID NO :19) : 
5'GAATTCCTCGAGAGATCTCTGCAGCCCGGGTTTTTATAGCTAATTAGTC
ATTTTTTCGTAAGTAAGTATTTTTATTTAA 3'
pour isoler un fragment PCR de 438 pb (fragment D).

Une réaction PCR a été réalisée à partir de la matrice constituée par l'ADN génomique du virus canarypox et avec les oligonucléotides suivants:
FC124 (78 mer) (SEQ ID NO :20) : 
5'CCCGGGCTGCAGAGATCTCTCGAGGAATTCTTTTTATTGATTAACTAGT
CAAATGAGTATATATAATTGAAAAAGTAA 3'
et C6D1 (45 mer) (SEQ ID NO :21) :
   5' GATGATGGTACCTTCATAAATACAAGTTTGATTAAACTTAAGTTG 3'
pour isoler un fragment PCR de 1216 pb (fragment E).

Les fragments D et E ont été hybridés ensemble pour servir de matrice à une réaction PCR réalisée avec les oligonucléotides C6A1 (SEQ ID NO :18) et C6D1 (SEQ ID NO :21) pour générer un fragment PCR de 1642 pb. Ce fragment a été digéré par les enzymes de restriction Sacl et Kpnl, pour isoler, après électrophorèse en gel d'agarose, un fragment SacI-KpnI de 1625 pb. Ce fragment a été ligaturé avec le vecteur pBlueScript® II SK+ (Stratagene, La Jolla, CA, USA, Cat # 212205), préalablement digéré par les enzymes de restriction SacI et KpnI, pour donner le plasmide pFC117. La séquence de ce plasmide a été vérifiée par séquençage. Ce plasmide contient 370 pb de séquences situées en amont du COL C6L (« bras flanquant gauche C6 »), un signal vaccine d'arrêt précoce de transcription, des codons stops dans les 6 phases de lecture, un site de clonage multiple contenant les sites de restriction Smal, Pstl, BglII, XhoI et EcoRI, et enfin 1156 pb de séquences situées en aval du COL C6L (« bras flanquant droit C6 »).

Le plasmide pMPIVC (Schmitt J. F. C. et al., J. Virol., 1988, 62, 1889-1897 ; Saiki R. K. et al., Science, 1988, 239, 487-491) a été utilisé comme matrice pour amplifier la séquence complète du promoteur vaccine I3L avec les oligonucléotides suivants :
FC112 (33 mer) (SEQ ID NO :22):
5' AAACCCGGGCGGTGGTTTGCGATTCCGAAATCT 3'
et FC113 (43 mer) (SEQ ID NO :23) :
5' AAAAGAATTCGGATCCGATTAAACCTAAATAATTGTACTTTGT 3'
pour amplifier un fragment PCR de 151 pb. Ce fragment a été digéré par les enzymes de restriction Smal et EcoRI pour isoler, après électrophorèse en gel d'agarose, un fragment de restriction Smal-EcoRI d'environ 136 pb. Ce fragment a alors été ligaturé avec le plasmide pFC117, préalablement digéré par Smal et EcoRI, pour donner le plasmide pFC118.

### Exemple 10 : construction du virus recombiné vCP1719

Les fragments C et D (exemple 5) ont été ligaturés avec le plasmide pFC116 (exemple 8) préalablement digéré par les enzymes de restriction Pstl et BglII pour donner le plasmide pFC119.

Le fragment E (exemple 6) a été ligaturé avec le plasmide pFC118 (exemple 9) préalablement digéré par les enzymes de restriction PstI et BglII pour donner le plasmide pFC120.

Le plasmide pFC120 a été linéarisé par Notl, puis transfecté dans des cellules primaires d'embryons de poulets infectées avec du virus canarypox (souche ALVAC) selon la technique de précipitation au phosphate de calcium précédemment décrite (Panicali et Paoletti Proc. Nat. Acad. Sci. 1982. 79. 4927-4931 ; Piccini et al. In Methods in Enzymology. 1987. 153. 545-563. Eds. Wu R. and Grossman L. Academic Press). Des plages positives ont été sélectionnées sur la base d'une hybridation avec une sonde radiomarquée spécifique de la séquence nucléotidique de la protéine tat. Ces plages ont subi 4 cycles successifs de sélection/purification de plages jusqu'à ce qu'une population pure ait été isolée. Une plage représentative de la recombinaison *in vitro* entre le plasmide donneur pFC120 et le génome du virus canarypox ALVAC a alors été amplifiée et le stock de virus recombiné obtenu a été désigné vCP1719.

Optionnellement, les virus recombinés obtenus ont été utilisés pour une deuxième transfection dans des cellules primaires d'embryons de poulets en présence de plasmide pFC119 linéarisé par Notl, selon la technique de précipitation au phosphate de calcium. Des plages positives ont été sélectionnées sur la base d'une hybridation avec une sonde radiomarquée spécifique de la séquence nucléotidique de la protéine rev. Ces plages ont subi 4 cycles successifs de sélection/purification de plages jusqu'à ce qu'une population pure ait été isolée. Une plage représentative de la recombinaison *in vitro* entre les plasmides donneurs pFC119, pFC120 et le génome du virus canarypox ALVAC a alors été amplifiée et le stock de virus recombiné obtenu a été désigné vCP1720.

### Exemple 11 : Construction du plasmide pJP090

Du sang de chat a été récolté sur un tube contenant de l'EDTA par une prise de sang à la veine jugulaire. Les cellules mononucléées ont été récoltées par centrifugation sur un gradient de Ficoll, puis mises en culture en boîte de Petri de 60 mm de diamètre. Les cellules mononuclées de chat en culture ont alors été stimulés soit avec de la concanavaline A (conA) (concentration finale d'environ 5 µg/ml) soit avec de la phyto-hémagglutinine (PHA) (concentration finale d'environ 10 µg/ml). Après stimulation, les lymphoblastes « ConA » et « PHA » ont été récoltés par grattage des boîtes de culture, et l'ARN total de ces cellules a été extrait en utilisant le kit « mRNA isolation kit for White Blood Cells » (Boehringer Mannheim/Roche Cat # 1 934 325).

L'ARN total extrait des lymphocytes de chat stimulés par la ConA ou par la PHA a servi de matrice pour la synthèse du premier brin d'ADN complémentaire. Ce premier brin d'ADN complémentaire a été produit par élongation de l'oligonucléotide p(dT)15 (Boehringer Mannheim/Roche Cat # 814 270). L'ADN complémentaire simple brin obtenu a été ensuite utilisé comme matrice pour une réaction d'PCR avec les oligonucléotides suivants :
FC125 (48 mer) (SEQ ID N° 24) :
5' TTTTTTGCGGCCGCCACCATGTGGCTGCAGAACCTGCTTTTCCTGGGC 3'
et FC126 (50 mer) (SEQ ID N°25) :
5' TTTTTTGCGGCCGCTACGTATCACTTCTTGACTGGTTTCCAGCAGTCAAA 3'
pour amplifier un fragment PCR d'environ 473 paires de bases (pb). Ce fragment a été purifié par électrophorèse en gel d'agarose. Ce fragment a été digéré par Notl et le fragment Notl-Notl d'environ 453 pb ainsi obtenu a été ligaturé avec le plasmide pVR1012 (exemple 3), préalablement digéré avec Notl, pour donner le plasmide pJP090 (5365 pb). L'orientation de l'insert dans pJP090 a été vérifiée. Le fragment Notl-Notl cloné sur ce plasmide a été entièrement séquencé. Cette séquence (SEQ ID N° 26), qui code pour une protéine de 144 acides aminés (SEQ ID N° 27) est la cytokine GM-CSF féline.

### Exemple 12 : Production de vaccins ADN

Une solution d'ADN contenant le plasmide pPB371 (exemple 3) est concentrée par précipitation éthanolique comme décrit dans Sambrook et al (1989). Le culot d'ADN est repris par une solution de NaCl 1,8% de façon à obtenir une concentration de 1 mg/ml. Une solution de DMRIE-DOPE à 0,75 mM est préparée par reprise d'un lyophilisat de DMRIE-DOPE par un volume adapté d'H₂O stérile.

La formation des complexes ADN plasmidique-lipide est réalisée par dilution à parties égales de la solution à 0.75 mM de DMRIE-DOPE (1:1) par la solution d'ADN à 1 mg/ml dans NaCl 1,8%. La solution d'ADN est introduite progressivement à l'aide d'une aiguille sertie 26G le long de la paroi du flacon contenant la solution de lipide cationique de façon à éviter la formation de mousse. On procède à une agitation douce dès que les deux solutions sont mélangées. On obtient en final une composition comprenant 0,375 mM de DMRIE-DOPE et 500 µg/ml de plasmide.

Il est souhaitable que l'ensemble des solutions utilisées soient à température ambiante pour l'ensemble des opérations décrites ci-dessus. On laisse la complexation ADN/DMRIE-DOPE se mettre en place à température ambiante pendant 30 minutes avant de procéder à l'immunisation des animaux.

Des vaccins ADN peuvent également être produits avec des solutions d'ADN contenant les plasmides pPB374 (exemple 4), pPB375 (exemple 5), pPB383 (exemple 6), pJP090 (exemple 11) ou des mélanges d'au moins deux de ces 5 plasmides selon la technique décrite dans le présent exemple.

### Exemple 13 : Tests d'expression in vitro

L'expression des protéines FIV est testée pour chaque construction par les méthodes classiques d'immunofluorescence indirecte et de Western Blot.

Ces tests sont effectués sur boîtes de Pétri contenant les cellules CHO cultivées en monocouches et transfectées par des plasmides ou contenant les cellules CEF cultivées en monocouches et infectées par des virus recombinés.

Les protéines FIV sont détectées par utilisation de sérums de chats infectés et d'anti-sérums marqués.

La taille des fragments obtenus après migration sur gel d'agarose est comparée à celles attendues.

### Exemple 14 : Efficacité sur animaux

Des chats Hillgrove (Biological Laboratories Europe Ltd.) EOPS et sans anticorps anti-FIV, approximativement âgés de 12 semaines sont randomisés en trois groupes de 6 animaux.

Les chats du premier groupe (groupe A) sont vaccinés à J0 et J28 par administration intramusculaire d'1 ml de mélange de plasmides pPB371 (exemple 3) et pPB374 (exemple 4), puis administration de rappel à J56 par voie intramusculaire d'1 ml de virus recombiné vCP255 (exemple 7) à un titre de 10^{8,0} TCID₅₀/ml.

Les chats du deuxième groupe (groupe B) sont vaccinés à J0 et J28 par administration intramusculaire d'1 ml de mélange des plasmides pPB371 et pPB374, formulé avec du DMRIE-DOPE (exemple 12), puis administration de rappel à J56 par voie intramusculaire d'1 ml de virus recombiné vCP255 (exemple 7) à un titre de 10^{8,0} TCID₅₀/ml.

La concentration en ADN total dans les vaccins ADN est de 200 µg/ml, soit 100 µg/ml pour chaque plasmide contenu dans le mélange.

Le rapport molaire lipide/ADN pour les vaccins ADN formulés avec du DMRIE-DOPE est de 0,25.

Le troisième groupe (contrôles) est le groupe témoin (pas de vaccination, épreuve à J84).

Tous les chats sont éprouvés à J84 par administration intra-péritonéale de 1 ml de virus FIV pathogène (souche Petaluma) à un titre de 25 CID₅₀/ml (CID étant dose infectieuse 50% chez le chat).

Sont observés d'une part la virémie appréciée par réisolement viral et PCR, la réponse en anticorps.

Réisolement viral à partir de la semaine 4 après épreuve à la semaine 16 (nombre d'animaux présentant une virémie positive) :

| Groupes | réisolement viral | PCR |
|---|---|---|
| Groupe A | 2/6 | 2/6 |
| Groupe B | 3/6 | 2/6 |
| Contrôles | 6/6 | 5/6 |

Le réisolement viral se réalise par co-culture d'environ 5 10° cellules mononucléées du sang périphérique (PBMC) avec environ 10⁶ cellules MYA-1 en milieu RPMI 1640 pendant 21 jours. La présence d'ADN proviral FIV présent dans les cellules PBMC est identifiée par PCR.

On observe une absence de virémie chez 67% des animaux du groupe A et 50% du groupe B.

Réponse cellulaire le jour de l'épreuve et 4 semaines après épreuve (nombre d'animaux présentant une réponse CTL positive) :

| Antigène détecté | Gag | Gag | Env | Env |
|---|---|---|---|---|
| | Semaine 0 | Semaine 4 | Semaine 0 | Semaine 4 |
| Groupe A | 1/6 | 4/6 | 0/6 | 2/6 |
| Groupe B | 2/6 | 6/6 | 2/6 | 0/6 |
| Contrôles | 0/5* | 2/6 | 0/5* | 0/6 |

| | | | | |
|---|---|---|---|---|
| * : les prélèvements n'ont pas pu être réalisés sur 1 animal. | | | | |

Des fibroblastes cutanés sont prélevés par biopsie chez chacun des chats. Les fibroblastes sont marqués au ⁵¹Cr et ensuite infectés par un recombinant vaccine exprimant soit env soit gag en présence de PBMC provenant de chacun des chats. La réponse cytotoxique (CTL) est mesurée par la libération de ⁵¹Cr.

On observe que la vaccination stimule (effet priming) la réponse cytotoxique pour les groupes d'animaux vaccinés, particulièrement pour gag.

Réponse humorale après épreuve (nombre d'animaux ayant une réponse sérologique positive en ELISA anti-TM):

| Semaine | 0 | 2 | 4 | 8 | 12 | 16 |
|---|---|---|---|---|---|---|
| Groupe A | 0/6 | 3/6 | 4/6 | 3/6 | 3/6 | 5/6 |
| Groupe B | 0/6 | 3/6 | 5/6 | 5/6 | 4/6 | 6/6 |
| Contrôles | 0/6 | 0/6 | 0/6 | 3/6 | 5/6 | 5/6 |

Il s'agit d'un ELISA pour quantifier les anticorps en utilisant un peptide correspondant à l'épitope majeur de la protéine transmembranaire (TM).

La vaccination stimule (effet priming) la réponse immunitaire humorale.

### SEQUENCE LISTING

<110> Merial<120> Vaccination contre le virus de l'immunodéficience féline<130> FIV prime/boost<160> 25 <170> PatentIn version 3.1<210> 1<211> 36<212> DNA<213> Artificial<400> 1
   ttttttctgc agcaataaga atggcagaag gatttg 36
<210> 2<211> 36<212> DNA<213> Artificial<400> 2
   tcgcacctga aacatctcga gtgtttccac atgtat 36
<210> 3<211> 36<212> DNA<213> Artificial<400> 3
   atacatgtgg aaacactcga gatgtttcag gtgcga 36
<210> 4<211> 54<212> DNA<213> Artificial<400> 4
   ttttttggat cccccgggct gcaggaattc tgagatactt catcattcct cctc 54
<210> 5<211> 37<212> DNA<213> Artificial<400> 5
   tttgtcgaca aggtaggaga gattctacag caacatg 37
<210> 6<211> 40<212> DNA<213> Artificial<400> 6
   tttgcggccg cgttattgag ccattactaa cctaatattg 40
<210> 7<211> 48<212> DNA<213> Artificial<400> 7
   tttttacctg catttccttc ttccagtttt acctcttgaa tttcgttc 48
<210> 8<211> 48<212> DNA<213> Artificial<400> 8
   tttactggaa gaaggaaatg caggtaaaag gaaaagacaa agaagaag 48
<210> 9<211> 36<212> DNA<213> Artificial<400> 9
   tttagatctt tagtccataa gcattctttc tatttc 36
<210> 10<211> 29<212> DNA<213> Artificial<400> 10
   tttctgcaga tggaagacat aatagtatt 29
<210> 11<211> 32<212> DNA<213> Artificial<400> 11
   tttagatctc taagcagtag ttattgataa tg 32
<210> 12<211> 42<212> DNA<213> Artificial<400> 12
   atcatcgagc tccagctgta attcatggtc gaaaagaagt gc 42
<210> 13<211> 79<212> DNA<213> Artificial<400> 13 
gaattcctcg agagatctct gcagcccggg tttttatagc taattagtca ttttttgaga 60

gtaccacttc agctacctc 79
<210> 14<211> 78<212> DNA<213> Artificial<400> 14 
cccgggctgc agagatctct cgaggaattc tttttattga ttaactagtc attataaaga 60
tctaaaatgc ataatttc 78
<210> 15<211> 45<212> DNA<213> Artificial<400> 15
   gatgatggta ccgtaaacaa atataatgaa aagtattcta aacta 45
<210> 16<211> 34<212> DNA<213> Artificial<400> 16
   aaacccgggt tctttattct atacttaaaa agtg 34
<210> 17<211> 43<212> DNA<213> Artificial<400> 17
   aaaagaattc gtcgactacg atacaaactt aacggatatc gcg 43
<210> 18<211> 42<212> DNA<213> Artificial<400> 18
   atcatcgagc tcgcggccgc ctatcaaaag tcttaatgag tt 42
<210> 19<211> 79<212> DNA<213> Artificial<400> 19 
gaattcctcg agagatctct gcagcccggg tttttatagc taattagtca ttttttcgta 60
agtaagtatt tttatttaa 79
<210> 20<211> 78<212> DNA<213> Artificial<400> 20 
cccgggctgc agagatctct cgaggaattc tttttattga ttaactagtc aaatgagtat 60
atataattga aaaagtaa 78
<210> 21<211> 45<212> DNA<213> Artificial<400> 21
   gatgatggta ccttcataaa tacaagtttg attaaactta agttg 45
<210> 22<211> 33<212> DNA<213> Artificial<400> 22
   aaacccgggc ggtggtttgc gattccgaaa tct 33
<210> 23<211> 43<212> DNA<213> Artificial<400> 23
   aaaagaattc ggatccgatt aaacctaaat aattgtactt tgt 43
<210> 24<211> 48<212> DNA<213> Artificial<400> 24
   ttttttgcgg ccgccaccat gtggctgcag aacctgcttt tcctgggc 48
<210> 25<211> 50<212> DNA<213> Artificial<400> 25
   ttttttgcgg ccgctacgta tcacttcttg actggtttcc agcagtcaaa 50

## Revendications

1. Kit pour l'induction d'une réponse immunitaire dirigée contre FIV chez un chat, comprenant, conditionnés séparément :
- une première composition immunogène comprenant, dans un véhicule ou excipient pharmaceutiquement acceptable, un ou des plasmide(s) exprimant *in vivo* env et gag/pro de FIV,
- une seconde composition immunogène comprenant, dans un véhicule ou excipient pharmaceutiquement acceptable, un ou des vecteur(s) viral(aux) atténué(s) exprimant *in vivo* env et gag/pro de FIV, dans laquelle le ou les vecteur(s) viral(aux) atténué(s) sont un ou des virus canarypox atténué(s) ou un ou des virus fowlpox atténué(s).

2. Kit selon la revendication 1, dans lequel la première composition immunogène et/ou la seconde composition immunogène exprime *in vivo* tat de FIV.

3. Kit selon la revendication 1 ou 2, dans lequel le ou les vecteur(s) viral(aux) atténué(s) sont un ou des virus canarypox atténué(s).

4. Kit selon l'une quelconque des revendications 1 à 3, comprenant des compositions immunogènes pour induire une réponse immunitaire dirigée contre FIV chez un chat ou chez plusieurs chats.

5. Kit selon l'une quelconque des revendications 1 à 4, comprenant deux doses de composition immunogène à base de plasmides pour une dose de composition immunogène à base de vecteurs viraux atténués.

6. Kit selon l'une quelconque des revendications 1 à 5, dans lequel la composition immunogène à base de plasmides et/ou la composition immunogène à base de vecteurs viraux atténués comporte un adjuvant.

7. Kit selon la revendication 6, dans lequel l'adjuvant est une cytokine.

8. Kit selon la revendication 7, dans lequel la cytokine est un GM-CSF félin.

9. Kit selon l'une quelconque des revendications 1 à 8, dans lequel la seconde composition immunogène est pour une administration en rappel de la première composition immunogène.

10. Kit selon l'une quelconque des revendications 1 à 9, dans lequel la première et la seconde composition immunogène est pour une administration par voie intramusculaire.

11. Utilisation d'une part d'un ou de plusieurs plasmide(s) exprimant *in vivo* env et gag/pro de FIV, pour la production d'une première composition immunogène comprenant le ou les plasmide(s) et un véhicule ou excipient pharmaceutiquement acceptable, pour une primo-administration à un chat, et d'autre part un ou des vecteur(s) viral(aux) atténué(s) exprimant *in vivo* env et gag/pro de FIV, pour la production d'une seconde composition immunogène comprenant le ou les vecteur(s) viral(aux) atténué(s) et un véhicule ou excipient pharmaceutiquement acceptable, pour une administration en rappel à un intervalle de 3 à 6 semaines au même chat,
dans laquelle
l'administration de la première et la seconde composition immunogène se fait par voie intramusculaire, et
le ou les vecteur(s) viral(aux) atténué(s) sont un ou des virus canarypox atténué(s) ou un ou des virus fowlpox atténué(s).

12. Utilisation d'un ou de plusieurs plasmide(s) exprimant *in vivo* env et gag/pro de FIV, pour la production d'une composition immunogène comprenant le ou les plasmide(s) et un véhicule ou excipient pharmaceutiquement acceptable pour l'induction d'une réponse immunitaire dirigée contre FIV chez un chat, destinée à être administrée par voie intramusculaire au chat en primo-administration, le rappel étant effectué après 3 à 6 semaines à l'aide d'une composition immunogène comprenant un ou plusieurs vecteur(s) viral(aux) atténué(s) exprimant *in vivo* env et gag/pro de FIV et un véhicule ou excipient pharmaceutiquement acceptable,
dans laquelle ie ou les vecteur(s) viral(aux) àtténué(s) sont un ou des virus canarypox atténué(s) ou un ou des virus fowlpox atténué(s).

13. Utilisation d'un ou plusieurs vecteur(s) viral(aux) atténué(s)exprimant *in vivo* env et gag/pro de FIV, pour la production d'une composition immunogène comprenant le ou les vecteur(s) viral(aux) atténué(s) et un véhicule ou excipient pharmaceutiquement acceptable, pour l'induction d'une réponse immunitaire dirigée contre FIV chez un chat, destinée à être administrée par voie intramusculaire au chat en rappel à un intervalle de 3 à 6 semaines d'une composition immunogène comprenant un ou plusieurs plasmide(s) exprimant *in vivo* env et gag/pro de FIV et un véhicule ou excipient pharmaceutiquement acceptable,
dans laquelle le ou les vecteur(s) viral(aux) atténué(s) sont un ou des virus canarypox atténué(s) ou un virus fowlpox atténué(s).

14. Utilisation selon l'une quelconque des revendications 11 à 13, dans laquelle le ou les plasmide(s) et/ou le ou les vecteur(s) viral(aux) atténué(s) comprennent et expriment *in vivo* un polynucléotide codant pour tat de FIV.

15. Utilisation selon la revendication 14, dans laquelle le ou les vecteur(s) viral(aux) atténué(s) sont un ou des virus canarypox atténué(s).

16. Utilisation selon la revendication 15, dans laquelle les deux compositions immunogènes sont pour une administration à un intervalle de l'ordre de 4 semaines.

17. Utilisation selon l'une quelconque des revendications 11 à 16, dans laquelle la composition immunogène à base de plasmides et/ou la composition immunogène à base de vecteurs viraux atténués comporte un adjuvant.

18. Utilisation selon la revendication 17, dans laquelle l'adjuvant est une cytokine.

19. Utilisation selon la revendication 18, dans laquelle la cytokine est un GM-CSF félin.

## Claims

1. A kit for inducing an immune response directed against FIV in a cat, comprising, packaged separately:
- a first immunogenic composition comprising, in a pharmaceutically acceptable vehicle or excipient, one or several plasmid(s) expressing, *in vivo* FIV env and gag/pro,
- a second immunogenic composition comprising, in a pharmaceutically acceptable vehicle or excipient, one or several attenuated viral vector(s) expressing *in vivo* FIV env and gag/pro,
wherein the one or several attenuated viral vector(s) are one or several attenuated canarypox virus(es) or one or several attenuated fowlpox virus(es).

2. The kit according to claim 1, wherein the first immunogenic composition and/or the second immunogenic composition expresses *in vivo* a FIV tat.

3. The kit according claim 1 or 2, wherein the attenuated viral vector(s) are one or several attenuated canarypox virus(es).

4. The kit according to any one of claims 1 to 3, wherein the kit comprises doses of immunogenic composition to induce an immune response directed against FIV in a cat or several cats.

5. The kit according to any one of claims 1 to 4, wherein the kit comprises two doses of plasmid-based immunogenic composition per dose of attenuated viral vector-based immunogenic composition.

6. The kit according to any one of claims 1 to 5, wherein the plasmid-based immunogenic composition and/or the attenuated viral vector-based immunogenic composition comprise an adjuvant.

7. The kit according to claim 6, wherein the adjuvant is a cytokine.

8. The kit according to claim 7 wherein the cytokine is a feline GM-CSF.

9. The kit according to any one of claims 1 to 8, wherein the second immunogenic composition is for administration as a booster of the first immunogenic composition.

10. The kit according to any one of claim 1 to 9, wherein the first and second immunogenic composition is for intramuscular administration.

11. Use, firstly, of one or several plasmid(s) expressing, *in vivo* FIV env and gag/pro, for producing a first immunogenic composition comprising the one or several plasmid(s) and a pharmaceutically acceptable vehicle or excipient, for administration to a cat as a primary administration and, secondly, of one or several attenuated viral vector(s) expressing *in vivo* FIV env and gag/pro, for producing a second immunogenic composition comprising the attenuated viral vector(s) and a pharmaceutically acceptable vehicle or excipient, for administration 3 to 6 weeks apart to the same cat as a booster,
wherein
the administration of first and second immunogenic composition is intramuscular, and
the one or several attenuated viral vector(s) are one or several attenuated canarypox virus(es) or one or several attenuated fowlpox virus(es).

12. Use of one or several plasmid(s) expressing *in vivo* FIV env and gag/pro, for producing an immunogenic composition comprising the one or several plasmid(s) and a pharmaceutically acceptable vehicle or excipient, for inducing an immune response directed against FIV in a cat, to be administered intramuscularly to the cat as a primary administration, whereby a booster is effected after 3 to 6 weeks using an immunogenic composition comprising one or several attenuated viral vector(s) expressing *in vivo* FIV env and gag/pro and a pharmaceutically acceptable vehicle or excipient,
wherein the one or several attenuated viral vector(s) are one or several attenuated canarypox virus(es) or one or several attenuated fowlpox virus(es).

13. Use of one or several attenuated viral vector(s) expressing, *in vivo* FIV env and gag/pro, for producing an immunogenic composition comprising the one or several attenuated viral vector(s) and a pharmaceutically acceptable vehicle or excipient, for inducing an immune response directed against FIV in a cat, to be administered intramuscularly to a cat as a booster 3 or 6 weeks apart of an immunogenic composition comprising one or several plasmid(s) expressing, *in vivo,* FIV env and gag/pro and a pharmaceutically acceptable vehicle or excipient,
wherein the one or several attenuated viral vector(s) are one or several attenuated canarypox virus(es) or one or several attenuated fowlpox virus(es).

14. Use according to any one of claims 11 to 13, wherein the plasmid(s) and/or the attenuated viral vector(s) express *in vivo* FIV tat.

15. Use according to claim 14, wherein the attenuated viral vector(s) are one or several attenuated canarypox virus(es).

16. Use according to claim 15, wherein the two immunogenic compositions are for administration about 4 weeks apart.

17. Use according to any one of claims 11 to 16, wherein the plasmid-based immunogenic composition and/or the attenuated viral vector-based immunogenic composition comprise an adjuvant.

18. Use according to claim 17, wherein the adjuvant is a cytokine.

19. Use according to claim 18, wherein the cytokine is a feline GM-CSF.

## Patentansprüche

1. Kit zur Induktion einer Immunantwort, die gegen FIV bei einer Katze gerichtet ist, umfassend, einzeln verpackt:
- eine erste immunogene Zusammensetzung, die in einem pharmazeutisch verträglichen Träger oder Excipienten ein Plasmid oder Plasmide umfasst, das/die *in vivo* FIV-env und -gag/pro expimiert/exprimieren,
- eine zweite immunogene Zusammensetzung, die in einem pharmazeutisch verträglichen Träger oder Excipienten einen abgeschwächten viralen Vektor oder abgeschwächte virale Vektoren umfasst, der/die *in vivo* FIV-env und -gag/pro exprimiert/exprimieren, wobei der oder die abgeschwächte(n) virale(n) Vektor(en) ein abgeschwächtes Kanarienpockenvirus oder abgeschwächte Kanarienpockenviren oder ein abgeschwächtes Geflügelpockenvirus oder abgeschwächte Geflügelpockenviren ist/sind.

2. Kit nach Anspruch 1, wobei die erste immunogene Zusammensetzung und/oder die zweite immunogene Zusammensetzung *in vivo* FIV-tat exprimiert/exprimieren.

3. Kit nach Anspruch 1 oder 2, wobei der oder die abgeschwächte(n) virale(n) Vektor(en) ein abgeschwächtes Kanarienpockenvirus oder abgeschwächte Kanarienpockenviren ist/sind.

4. Kit nach einem der Ansprüche 1 bis 3, der immunogene Zusammensetzungen zur Induktion einer gegen FIV gerichteten Immunantwort in einer Katze oder mehreren Katzen umfasst.

5. Kit nach einem der Ansprüche 1 bis 4, der zwei Dosen einer immunogenen Zusammensetzung auf der Basis von Plasmiden auf eine Dosis einer immunogenen Zusammensetzung auf der Basis von abgeschwächten viralen Vektoren umfasst.

6. Kit nach einem der Ansprüche 1 bis 5, wobei die immunogene Zusammensetzung auf der Basis von Plasmiden und/oder die immunogene Zusammensetzung auf der Basis von abgeschwächten viralen Vektoren ein Adjuvans umfasst.

7. Kit nach Anspruch 6, wobei das Adjuvans ein Cytokin ist.

8. Kit nach Anspruch 7, wobei das Cytokin ein felines GM-CSF ist.

9. Kit nach einem der Ansprüche 1 bis 8, wobei die zweite immunogene Zusammensetzung für die Verabreichung zur Verstärkung der ersten immunogenen Zusammensetzung bestimmt ist.

10. Kit nach einem der Ansprüche 1 bis 9, wobei die erste und die zweite immunogene Zusammensetzung zur intramuskulären Verabreichung bestimmt sind.

11. Verwendung von einerseits einem oder mehreren Plasmiden, das/die *in vivo* FIV-env und -gag/pro exprimiert/exprimieren, zur Herstellung einer ersten immunogenen Zusammensetzung, die das oder die Plasmid(e) und einen pharmazeutisch verträglichen Träger oder Excipienten umfasst, für eine Erstverabreichung an eine Katze, und von andererseits einem abgeschwächten viralen Vektor oder von abgeschwächten viralen Vektoren, der/die *in vivo* FIV-env und -gag/pro exprimiert/exprimieren, zur Herstellung einer zweiten immunogenen Zusammensetzung, die den abgeschwächten viralen Vektor oder die abgeschwächten viralen Vektoren und einen pharmazeutisch verträglichen Träger oder Excipienten umfasst, für eine Verstärkungsverabreichung in einem Zeitraum von 3 bis 6 Wochen an dieselbe Katze,
wobei die Verabreichung der ersten und der zweiten immunogenen Zusammensetzung intramuskulär erfolgt, und
der oder die abgeschwächte(n) virale(n) Vektor(en) ein abgeschwächtes Kanarienpockenvirus oder abgeschwächte Kanarienpockenviren oder ein abgeschwächtes Geflügelpockenvirus oder abgeschwächte Geflügelpockenviren ist/sind.

12. Verwendung eines Plasmids oder mehrerer Plasmide, das/die *in vivo* FIV-env und -gag/pro exprimiert/exprimieren, zur Herstellung einer immunogenen Zusammensetzung, die das oder die Plasmid(e) und einen pharmazeutisch verträglichen Träger oder Excipienten umfasst, für die Induktion einer gegen FIV gerichteten Immunantwort bei einer Katze, und die zur intramuskulären Verabreichung als Erstverabreichung an eine Katze bestimmt ist, wobei die Verstärkungsverabreichung nach 3 bis 6 Wochen mittels einer immunogenen Zusammensetzung erfolgt, die einen abgeschwächten viralen Vektor oder mehrere abgeschwächten viralen Vektoren, der/die *in vivo* FIV-env und -gag/pro emprimiertlexprimieren, und einen pharmazeutisch verträglichen Träger oder Excipienten umfasst,
wobei der oder die abgeschwächte(n) virale(n) Vektor(en) ein abgeschwächtes Kanarienpockenvirus oder abgeschwächte Kanarienpockenviren oder ein abgeschwächtes Geflügelpockenvirus oder abgeschwächte Geflügelpockenviren ist/sind.

13. Verwendung eines abgeschwächten viralen Vektors oder mehrerer abgeschwächter viralen Vektoren, der/die *in vivo* FIV-env und -gag/pro exprimiert/exprimieren, zur Herstellung einer immunogenen Zusammensetzung, die den oder die abgeschwächten viralen Vektoren und einen pharmazeutisch verträglichen Träger oder Excipienten umfasst, für die Induktion einer gegen FIV gerichteten Immunantwort bei einer Katze, und die dazu bestimmt ist, in einem Zeitraum von 3 bis 6 Wochen intramuskulär an eine Katze zur Verstärkung einer immunogenen Zusammensetzung verabreicht zu werden, die ein oder mehrere Plasmide, das/die *in vivo* FIV-env und -gag/pro exprimiert/exprimieren, und einen pharmazeutisch verträglichen Träger oder Excipienten umfasst,
wobei der oder die abgeschwächte(n) virale(n) Vektor(en) ein abgeschwächtes Kanarienpockenvirus oder abgeschwächte Kanarienpockenviren oder ein abgeschwächtes Geflügelpockenvirus oder abgeschwächte Geflügelpockenviren ist/sind.

14. Verwendung nach einem der Ansprüche 11 bis 13, wobei das oder die Plasmid(e) und/oder der oder die abgeschwächte(n) virale(n) Vektor(en) ein FIV-tat codierendes Polynucleotid umfassen und *in vivo* exprimieren.

15. Verwendung nach Anspruch 14, wobei der oder die abgeschwächte(n) virale(n) Vektor(en) ein abgeschwächtes Kanarienpockenvirus oder abgeschwächte Kanarienpockenviren ist/sind.

16. Verwendung nach Anspruch 15, wobei die beiden immunogenen Zusammensetzungen für eine Verabreichung in einem Zeitraum von ungefähr 4 Wochen bestimmt sind.

17. Verwendung nach einem der Ansprüche 11 bis 16, wobei die immunogene Zusammensetzung auf der Basis von Plasmiden und/oder die immunogene Zusammensetzung auf der Basis von abgeschwächten viralen Vektoren ein Adjuvans umfasst.

18. Verwendung nach Anspruch 17, wobei das Adjuvans ein Cytokin ist.

19. Verwendung nach Anspruch 18, wobei das Cytokin ein felines GM-CSF ist.
